(19) Europäisches Patentamt
European Patent Office
Office européen des brevets

(11) EP 3 228 710 B1

(12) EUROPÄISCHE PATENTSCHRIFT

(45) Veröffentlichungstag und Bekanntmachung des Hinweises auf die Patenterteilung:
**06.03.2019 Patentblatt 2019/10**

(51) Int Cl.:
***C12N 15/10*** *(2006.01)*

(21) Anmeldenummer: **17163035.3**

(22) Anmeldetag: **27.03.2017**

# (54) AUFREINIGUNG VON NUKLEINSÄURE AUS EINER NUKLEINSÄURE UND ENDOTOXIN ENTHALTENDEN PROBE

PURIFICATION OF NUCLEIC ACIDS FROM A SAMPLE COMPRISING NUCLEIC ACIDS AND ENDOTOXIN

PURIFICATION D'ACIDES NUCLÉIQUES D'UN ÉCHANTILLON COMPRENANT DES ACIDES NUCLÉIQUES ET DE L'ENDOTOXIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.04.2016 DE 102016106271**

(43) Veröffentlichungstag der Anmeldung:
**11.10.2017 Patentblatt 2017/41**

(73) Patentinhaber: **AXAGARIUS GmbH & Co. KG**
**52355 Düren (DE)**

(72) Erfinder:
- **Heymans, Stefan**
  **42657 Solingen (DE)**
- **Rosenbaum, Thorsten**
  **52428 Jülich (DE)**

(74) Vertreter: **Paul & Albrecht Patentanwälte PartG mbB**
**Stresemannallee 4b**
**41460 Neuss (DE)**

(56) Entgegenhaltungen:
**WO-A1-2008/104811 WO-A2-2005/111059**
**CN-B- 103 173 438**

- **RUI MA ET AL: "Removing endotoxin from plasmid samples by Triton X-114 isothermal extraction", ANALYTICAL BIOCHEMISTRY, ELSEVIER, AMSTERDAM, NL, Bd. 424, Nr. 2, 10. Februar 2012 (2012-02-10), Seiten 124-126, XP028406546, ISSN: 0003-2697, DOI: 10.1016/J.AB.2012.02.015 [gefunden am 2012-02-25]**

EP 3 228 710 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft ein Verfahren zur Aufreinigung von Nukleinsäuren, insbesondere von Plasmid DNA, aus einer Nukleinsäure enthaltenden biologischen Probe umfassend die folgenden Schritte:

a) Bereitstellen einer flüssigen Probe, enthaltend Nukleinsäure und Endotoxin jeweils in gelöster Form;
b) Abscheidung zumindest von Nukleinsäure und Endotoxin aus der flüssigen Probe heraus;
c) Waschen der im Schritt b) abgeschiedenen Bestandteile der flüssigen Probe zum wenigstens teilweisen Entfernen des Endotoxins mit mindestens einer Waschlösung, die

(i) mindestens eine Amin-Verbindung mit mindestens zwei Kohlenstoffatomen und mit einer Molmasse von ≤ 500 g/mol und;
(ii) mindestens ein von besagter Amin-Verbindung verschiedenes organisches Lösemittel, enthält und

einen pH-Wert im Bereich von pH 3,0 bis pH 8,5 aufweist, und;

Inlösungbringen der verbliebenen Nukleinsäure aus den gemäß Schritt c) gewaschenen, abgeschiedenen Bestandteilen mittels eines Lösungspuffers und Auffangen der gelösten Nukleinsäuren in einem separaten Auffang-Gefäß.

Endotoxine sind Lipopolysaccharide (LPS) und bilden einen Bestandteil der Zellwand gram negativer Bakterien, wie zum Beispiel von Escherichia coli.Letzteres Bakterium wird sehr häufig in biotechnologischen Verfahren eingesetzt, daher sind Endotoxine als Kontaminante in vielen biotechnologisch hergestellten Produkten vorhanden. In der Gentherapie und bei der Herstellung pharmazeutischer Zusammensetzungen können Endotoxine im lebenden Organismus(in vivo)überschießende Immunreaktionen mit potentiell fatalen Auswirkungen hervorrufen. Auch bei der Transfektion von Nukleinsäuren in Zellkulturen (in vitro) ist ein negativer Einfluss von Endotoxinen beschrieben.

Die Entfernung von Endotoxinen aus Präparationen biotechnologisch hergestellter biologischer Makromoleküle ist ein aus dem Stand der Technik bekanntes Problem. Endotoxine werden in vielen bekannten Verfahren zur DNA-Aufreinigung ebenfalls isoliert und sind eine häufige Kontaminante von Plasmidisolierungen. Zur DNA-Aufreinigung eingesetzte Methoden sind in der Regel chromatographische Verfahren wie Anionentauscher und Affinitätschromatographie oder eine Extraktion mit Detergentien, wobei hier insbesondere die Verwendung von Triton® X-114 (i.e. 1,1,3,3-Tetramethylbutyl)phenyl-polyethylenglykol) und Deoxycholsäuresalze hervorzuheben sind. In der WO 2007/115046 A1 wird zudem ein Verfahren beschrieben, in dem Endotoxine zur Entfernung enzymatisch abgebaut werden.

Diese im Stand der Technik beschriebenen Verfahren haben Nachteile, da sie aufwendig und kostspielig sind und eine Vielzahl an Arbeitsschritten erfordern. Nachteilig an der Ionentauscherchromatographie oder der Affinitätschromatographie als Reinigungsmethode ist, dass zur Aufbereitung des Eluats die eluierte DNA umständlich entsalzt, aufkonzentriert und vom Elutionsmittel befreit werden muss, bevor sie für die nachfolgende Weiterverarbeitung oder für nachfolgende Analysen zur Verfügung stehen kann. Darüber hinaus sind chromatographische Festphasen teuer in der Herstellung, was die lonentauscherchromatographie unwirtschaftlich macht.

Zudem ist aus dem Stand der Technik bekannt, dass eine vollständige Entfernung der Endotoxine für einein vitroTransfektion nicht zwingend erforderlich und auf sensitive Zelllinien beschränkt ist. Insofern gibt es einen Mangel an einer kostengünstigen, einfachen und schnellen Methode zur weitgehenden Endotoxinabreinigung.

Wenige Schriften beschreiben eine Methode zur Abreinigung von Endotoxinen durch Fällung mit Alkoholen, da sich Endotoxine und DNA sehr ähnlich verhalten. Alkohole wie Ethanol und Isopropanol fällen in den Verfahren des Standes der Technik sowohl DNA als auch Endotoxine gemeinsam aus.

In dem Verfahren gemäß WO 95/21178A1 wird anstelle von Ethanol Isoproanol als flüssige Phase einer Anionentauscherchromatographie eingesetzt. Durch den Austausch vom einwertigen Alkohol Ethanol mit dem ebenfalls einwertigen Alkohol Isopropanol wird eine verbesserte Transfektionseffizienz erreicht.

In der WO 99/063076 A1 wird die Entfernung von Endotoxinen mittels Ethanol als flüssige Phase im Rahmen einer Anionentauscherchromatographie beschrieben. Zudem wird der Einsatz von Triton® X-114 offenbart, das bekanntermaßen zur Abreinigung von Endotoxinen in dieser Applikation geeignet ist.

In der EP 0 407 037 B1 wird ein Aufreinigungsverfahren offenbart, das eine komplizierte und umfangreiche fraktionierte Fällung mit Ethanol umfasst. Darin wird zudem Deoxycholat als ein häufig genanntes und bekanntes Detergenz zur Endotoxinabreinigung verwendet. Das beschriebene Verfahren soll Endotoxine von bakteriellen Polysacchariden abtrennen, eine Nukleinsäureaufreinigung ist jedoch weder Ziel noch Thema des Verfahrens.

Gemäß Druckschrift WO 2005/003152 A1 lassen sich Proteinkomplexe mit Endotoxinen nach Bindung an eine chromatographische Oberfläche unter Zuhilfenahme von Alkandiolen trennen. Dieses Verfahren funktioniert nicht für die Aufreinigung von Plasmid-DNA, da ein Kationentauscher gefordert wird. Generell gilt die zuvor genannte Limitierung der direkten Nutzbarkeit von Ionentauschereluaten.

Der Einsatz basischer Verbindungen als Hilfsstoffe für die chromatographische Aufreinigung ist aus dem Stand der Technik bekannt.

In der Druckschrift US 6 699 386 B2 wird die Verwendung eines basischen Materials zur affinitätschromatographischen Abtrennung von Endotoxinen beschrieben, wobei das basische Material an eine feste Oberfläche gebunden vorliegt. Die Herstellung einer entsprechenden Aufreinigungssäule ist aufwändig und es müssen weitere Aufreinigungsschritte für die eluierten, von Endotoxinen befreiten Substanzen vorgenommen werden. Darüber hinaus ist die Kapazität einer affinitätschromatographischen Säule beschränkt und abhängig von der Menge an eingesetztem Material, was das Totvolumen und somit das Elutionsvolumen unnötig vergrößert.

In der Druckschrift US 7 714 111 B2 wird ein Waschpuffer zur Entfernung von Verunreinigungen beschrieben, der ein Argininderivat enthält. Das in der Druckschrift offenbarte Verfahren wird zur Aufreinigung eines monoklonalen Antikörpers, der über den Fc-Teil an eine Protein-A-Affinitätschromatographische Säule gebunden wurde, vorgeschlagen. Als Verunreinigung soll eine heterologe Gruppe von Stoffen von besagtem Antikörper abgetrennt werden. Darunter fallen neben Endotoxinen auch Nukleinsäuren. Für die Nukleinsäureaufreinigung ist dieses Verfahren folglich nicht zu verwenden.

In der CN 103173438 B wird ein Verfahren zu Isolierung von Plasmiden beschrieben, bei welchem Endotoxine aktiv entfernt werden. Dabei liegen ein niedermolekulares Amin und ein organisches Lösungsmittel in zwei getrennten Waschlösungen vor. Ferner werden die Endotoxine mittels Affinitätsmatrix, gezielt von weiteren, in Lösung befindlichen Plasmiden getrennt.Aus der WO 2005/111059 A2 ist ein Verfahren zur Entfernung von Endotoxinen aus Plasmid-DNA enthaltenden Proben der eingangs genannten Art bekannt. Bei diesen Verfahren kommt ein Kohlenhydrat-basiertes nicht-ionisches Detergenz zusammen mit einem Silika-Trägermaterial zum Einsatz. An dem bekannten Verfahren kann es als nachteilig empfunden werden, dass es beim Verwenden üblicher Silika-Membranen als Trägermaterial zu Verstopfungen kommen kann, insbesondere dann, wenn größere Mengen an Nukleinsäuren abgetrennt werden sollen. Zudem sind auch die Ausbeuten an Nukleinsäuren nicht immer zufriedenstellend und ebenso wenig die Abtrennleistung hinsichtlich der Endotoxine.

Die Aufgabe der vorliegenden Erfindung war es, ausgehend vom Stand der Technik ein wirtschaftlicheres und in der Durchführung vereinfachtes Verfahren zur Aufreinigung von Nukleinsäuren, insbesondere von Plasmid DNA, aus einer Probe, enthaltend Nukleinsäure und Endotoxin, bereitzustellen. Das Verfahren soll insbesondere die bei der Ionentauscherchromatographie notwendigen Aufarbeitungsschritte sparen, und zu einem verminderten Aufwand von Zeit und Ressourcen für die Bereitstellung einer Lösung von Nukleinsäure mit reduziertem Endotoxingehalt führen. Zudem soll das neue Verfahren zur Endotoxinentfernung einfach in bestehende Nukleinsäureaufreinigungskits integrierbar sein.

Die Aufgabe wird bei einem Verfahren der eingangs genannten Art dadurch gelöst, dass die Waschlösung gemäß Schritt c) die besagte organische Amin-Verbindung in einer Konzentration von 200 mmol/L bis 1000 mmol/L enthält, und

im Rahmen der wenigstens teilweisen Entfernung, jeweils bezogen auf die Gesamtmenge an Endotoxin in der flüssigen Probe, wenigstens 50 Gew.-% Endotoxin entfernt werden.

Bei dem erfindungsgemäßen Verfahren handelt es sich um ein einfaches "Bind-Wash-Elute"-Verfahren. Im Rahmen eines "Bind-Wash-Elute"-Verfahrens werden Nukleinsäuren in dem erfindungsgemäßen Verfahren gemeinsam mit kontaminierenden Endotoxinen mittels bekannter Verfahren aus der flüssigen Probe abgeschieden und die abgeschiedenen Bestandteile der Probe durch Einsatz einer speziell geeigneten Waschlösung von einem Großteil der kontaminierenden Endotoxine befreit. Mit dieser Methode kann auf schnelle und kostengünstige Weise eine deutliche Reduzierung der Endotoxine erreicht werden und die eluierte Nukleinsäue, insbesondere die eluierte DNA, ist je nach gewählter Methode für die Nukleinsäureabscheidung direkt in Nachfolgeapplikationen einsetzbar.

Ein Stoff ist "flüssig", wenn er bei den Durchführungsbedingungen des erfindungsgemäßen Verfahrens, insbesondere bei 20°C und 1013 mbar, als Flüssigkeit vorliegt.

Die Verwendung unbestimmter Artikel (eine, ein) oder bestimmter Artikel (der, die, das) ist - falls nicht im Einzelfall anderweitig gekennzeichnet - im Sinne der vorliegenden Anmeldung nicht als beschränkende Mengenangabe aufzufassen. Gemeint ist - sofern nicht im Einzelfall anderweitig gekennzeichnet - eine Anzahl von mindestens eins (i.e. ein oder mehrere).

Als Nukleinsäuren kommen RNA und DNA unterschiedlicher Kettenlänge in Frage, insbesondere mit mehr als fünfzehn Nukleotiden, wie beispielsweise einzel- und doppelsträngige bakterielle, virale, humane, tierische oder pflanzliche RNA oder DNA, insbesondere genomische DNA, mitochondriale DNA, Plasmide, mRNA, tRNA, rRNA, miRNA und andere kurze RNA-Spezies, insbesondere mit einer Kettenlänge von 15 bis 25 Nukleotiden. Unter "Nukleinsäure" wird erfindungsgemäß bevorzugt Desoxyribonukleinsäure (DNA), besonders bevorzugt Plasmid DNA, verstanden.

Eine chemische Verbindung gilt als "organisch", wenn sie mindestens ein Kohlenstoffatom und ein daran kovalent gebundenes Wasserstoffatom enthält.

Zur Herstellung der flüssigen Nukleinsäure- und Endotoxin-haltigen Probe wird eine biologische Probe verwendet. Als solche biologische Probe eignet sich jedwedes biologische Material, das neben Nukleinsäuren auch Endotoxin enthält, wie Bakterienkulturen, tierisches oder menschliches Gewebe, Gewebeteile, Körperflüssigkeiten wie Speichel, Sputum, Liquor, Vollblut, Serum oder Plasma. Bakterien, Hefen und andere Pilze oder Viren werden ebenso unter "Probenmaterial" verstanden, wie auch PCR-Amplifikationsreaktionen, die Primer und DNA Fragmente enthalten, oder Zellkulturüberstände. Probenmaterial kann auch Umwelt- oder Lebensmittelproben umfassen. Auch künstliches Probenmaterial, z.B. mit synthetisch oder invitro erzeugten Nukleinsäuren fällt unter den Anwendungsbereich der vorliegenden Erfindung.

Zur Bereitstellung einer flüssigen Probe, enthaltend Nukleinsäure und Endotoxin jeweils in gelöster Form, kann im Rahmen des Schritts a) des erfindungsgemäßen Verfahrens die biologische Probe zunächst aufgeschlossen, d.h. lysiert, werden um die Nukleinsäuren und Endotoxin aus dem Material freizusetzen. Der Aufschluss kann einen mechanischen, chemischen und/ oder enzymatischen Aufschluss umfassen. Der Aufschluss der biologischen Probe wird häufig durch eine geeignete Pufferchemie unterstützt, die beispielsweise Detergenzien beinhaltet. Geeignete Lysebedingungen sind dem Fachmann bekannt.

Im Schritt b) des erfindungsgemäßen Verfahrens wird die Nukleinsäure zusammen mit Endotoxin mittels eines beliebigen dem Fachmann bekannten Verfahrens aus der flüssigen Probe abgeschieden.

Das erfindungsgemäße Verfahren ist dabei nicht auf ein bestimmtes Prinzip der Abscheidung beschränkt. Dem Stand der Technik sind verschiedenartige Verfahren zu entnehmen, die angewendet werden können und dem Fachmann bekannt sind. Diese umfassen beispielsweise die Verwendung chaotroper Salze, die Verwendung anti-chaotroper Salze, Ausfällungen (z.B. Fällungen mit Polyethylenglycol oder niederen Alkoholen), Filtration, die Ausnutzung hydrophober Wechselwirkungen für die Nukleinsäurebindung an ein Trägermaterial und andere Verfahren.

Eine bevorzugte Form der Abscheidung ist dadurch gekennzeichnet, dass im Schritt b) zumindest Nukleinsäure und Endotoxin durch Bindung, insbesondere durch Adsorption oder Fällung, an eine Oberfläche eines festen Trägermaterials abgeschieden werden. Dabei ist es wiederum besonders bevorzugt, wenn die Probe im Schritt b) zur Abscheidung an das Trägermaterial mit einem Bindepuffer versetzt wird.

Der Bindepuffer enthält bevorzugt mindestens ein chaotropes Salz und/oder mindestens einen Monoalkohol mit bis zu 16 Kohlenstoffatomen.

Gemäß geltender Theorien zerstören die chaotropen Salze die geordnete Wasserstruktur um in Wasser gelöste Verbindungen. Chaotrope Salze sind demnach so definiert, dass sie Proteine denaturieren, die Löslichkeit unpolarer Substanzen in Wasser erhöhen sowie hydrophobe Wechselwirkungen zerstören. Es ist bekannt, dass Nukleinsäure in Gegenwart chaotroper Salze reversibel an Trägermaterialien, insbesondere an Silikate und an andere anorganische Trägermaterialien, bindet. Die chaotropen Salze bewirken hierbei eine Zerstörung der Hydrathülle der Nukleinsäuren und schaffen eine hydrophobe Mikroumgebung. Unter diesen Bedingungen binden sowohl Nukleinsäuren als auch Endotoxin an das feste Trägermaterial, während Proteine und andere Kontaminanten nicht binden und ausgewaschen werden. Die Stärke des chaotropen Charakters eines Salzes ist in der so genannten Hofmeister-Reihe beschrieben.

In dem Bindepuffer sind chaotropes Salz bevorzugt in einer Konzentration von 1 bis 6 mol/L enthalten.

Im Rahmen der vorliegenden Erfindung werden Natriumperchlorat, Natriumiodid, Guanidinium isothiocyanat, Guanidinium hydrochlorid, Kaliumthiocyanat, Guanidinium nitrat, Guanidinium carbonat, Harnstoff oder auch Kombination hiervon als chaotrope Salze bevorzugt verwendet.

Es ist erfindungsgemäß ganz besonders bevorzugt, wenn der Bindepuffer mindestens ein chaotropes Salz oder mindestens einen Monoalkohol mit bis zu 16 Kohlenstoffatomen oder ein organisches Polymer enthält.

Die Abscheidung von Nukleinsäure und Endotoxin aus der Probe kann wie zuvor ausgeführt wurde bevorzugt über eine Anlagerung der besagten Biomoleküle an ein festes Trägermaterial vorgenommen werden. Das feste Trägermaterial wird bevorzugt ausgewählt aus mineralischen Trägermaterialien, insbesondere Quarzfasern, Silica, Glas, Aluminiumoxid, Zeolith, Titandioxid, Zirkondioxid oder Kombinationen daraus. Diese Träger können in partikulärer Form vorliegen, vor allem als magnetische und/oder magnetisierbare Partikel, insbesondere Magnetbeads. Weiterhin kann das feste Trägermaterial in Form von Fasern, Schwämmen, Schäumen, insbesondere in Form von Fritten oder Membranen eingesetzt werden. In bevorzugter Weise ist das feste Trägermaterial als chromatographische Säule ausgestaltet. Dabei kann das feste Trägermaterial die Säulenpackung und/oder eine Membran darstellen, wozu insbesondere die oben genannten mineralischen Trägermaterialien verwendet werden.

Die Abscheidung von Nukleinsäure und Endotoxin an das feste Trägermaterial kann dadurch erzielt werden, dass die Probe mit dem festen Trägermaterial in Kontakt gebracht wird. Bei Einsatz magnetischer oder magnetisierbarer Partikel, insbesondere Magnetbeads, als Trägermaterial können diese auch direkt zur Probe gegeben werden. Mit dem Zusatz der Partikel kann zur Verbesserung der Bindebedingungen die Probe danach noch mit Bindepuffer (vide supra),versetzt werden und/oder die Abtrennung der beladenen Partikel durch Zentrifugieren, Filtration, Vakuumfiltration oder Magnetseparation unterstützt werden.

Werden mineralische Trägermaterialien, wie Silica, als festes Trägermaterial eingesetzt, kann die Probe bevorzugt zur Verbesserung der Bindebedingungen zuvor noch mit einem Bindepuffer (vide supra) versetzt werden. Es ist erfindungsgemäß besonders bevorzugt zur Abscheidung im Schritt b) des erfindungsgemäßen Verfahrens ein festes Trägermaterial enthaltend Silica in Kombination mit einem Bindepuffer (insbesondere den als bevorzugt gekennzeichneten Bindepuffern (vide supra),ganz besonders bevorzugt mit einem Bindepuffer, enthaltend mindestens ein chaotropes

Salz bzw. deren bevorzugte Ausführungsformen (vide supra)) zu verwenden.

Aufreinigungsverfahren mit einer der Abfolge von Bindung des Produktes, einem oder mehreren Waschschritten und schließlich der Elution des gereinigten Produktes werden als "Bind-Wash-Elute" Verfahren bezeichnet.

Üblicherweise werden für die "Bind-Wash-Elute"-Reinigung Zentrifugationssäulen verwendet, in denen als festes Trägermaterial Silica oder ein alternatives mineralisches Trägermaterial dient. Da die einzelnen Prozessierungsschritte, wie etwa das Binden der Nukleinsäuren, üblicherweise in kleinen Labortischzentrifugen stattfinden, werden die entsprechenden Säulen als "Minispin"-Säulen bezeichnet. Diese Säulen sind aus dem Stand der Technik grundsätzlich bekannt und betreffen kleine Säulen, welche beispielsweise in 1,5 mL Eppendorf Reaktionsgefäße eingesetzt und darin in einer Mikrozentrifuge verarbeitet werden können.

Es ist erfindungsgemäß ganz besonders bevorzugt, wenn in Schritt b) des erfindungsgemäßen Verfahrens das feste Trägermaterial in Form einer "Minispin"-Säule für die Anwendung in einer Laborzentrifuge ausgestaltet ist.

Das auf dem Trägermaterial abgeschiedene Gemisch aus Nukleinsäure und Endotoxin wird bevorzugt in der auf dem festen Trägermaterial gebundenen Form im Schritt c) des erfindungsgemäßen Verfahrens eingesetzt.

Als weitere bevorzugte Möglichkeit der Abscheidung gemäß Schritt b) können zumindest die Nukleinsäure und das Endotoxin aus der flüssigen Probe als Feststoff durch Fällung abgeschieden werden. Hierzu kann der flüssigen Probe ein Fällungsreagenz zugegeben werden, das Nukleinsäure und Endotoxin als Feststoff aus der flüssigen Probe ausfallen lässt. Das Fällungsreagenz enthält bevorzugt mindestens eine Verbindung ausgewählt aus Tripropylenglycol (bevorzugt in Kombination mit einem (C1-C4)-Alkohol (besonders bevorzugt Methanol, Ethanol, n-Propanol oder Isopropanol, ganz besonders bevorzugt Ethanol)), Polyethylenglycolen mit einem Molekulargewicht zwischen 300 bis 10000 g/mol, Polypropylenglycolen mit einem Molekulargewicht zwischen 300 bis 10000 g/mol, kationischen Detergentien (wie Hexadecyltrimethylammoniumbromid (CTAB), Pyridiniumsalze oder quartäre Ammoniumverbindungen mit einem langen, kettenförmigen Kohlenwasserstoffrest (C6- C18) und drei Resten, ausgewählt unter kurzen Kohlenwasserstoffresten (C1-C3) oder Wasserstoff), Ethanol, n-Propanol, Isopropanol oder Kombinationen daraus.

Wenn (gegebenenfalls unter Zugabe des Fällungsreagenzes) Nukleinsäure und Endotoxin als Feststoff aus der flüssigen Probe ausgefallen sind, ist es wiederum bevorzugt, die gefällte Nukleinsäure und das Endotoxin als Feststoff

i) durch Filtration auf der Oberfläche eines festen Trägermaterials zu sammeln, oder
ii) durch Zentrifugation zu verdichten,

und jeweils von der Mutterlösung abzutrennen.

Das Trägermaterial zur Sammlung des ausgefällten Feststoffes wird gemäß besonders bevorzugter Ausführungsform i) wiederum bevorzugt ausgewählt aus mineralischen Trägermaterialien, insbesondere aus Quarzfasern, Silicagel, Glas, Aluminiumoxid, Zeolith, Titandioxid, Zirkondioxid oder Kombinationen daraus.

Es ist erfindungsgemäß besonders bevorzugt, für die Filtration das feste Trägermaterial aus mineralischen Trägermaterialien, insbesondere aus Quarzfasern, Silicagel, Glas, Aluminiumoxid, Zeolith, Titandioxid, Zirkondioxid oder Kombinationen daraus, auszuwählen und für die Fällung von Nukleinsäure und Endotoxin mindestens ein Fällungsreagenz, insbesondere mindestens ein bevorzugt geeignetes Fällungsrearenz(vide supra),ganz besonders bevorzugt Tripropylenglycol (bevorzugt in Kombination mit einem (C1-C4)-Alkohol (besonders bevorzugt Methanol, Ethanol, n-Propanol oder Isopropanol)) einzusetzen. Ein ganz bevorzugtes Fällungsreagenz enthält Tripropylenglycol und Ethanol.

Wird der besagte Feststoff in einem Gefäß mittels Zentrifuge gemäß Alternative ii) verdichtet, so lagert sich der besagte Feststoff auf der Wandung des Gefäßes ab. Die überstehende Mutterlösung kann beispielsweise mit Hilfe einer Pipette abgetrennt werden.

Zurück bleibt gemäß Alternative i) und Alternative ii) der abgeschiedene besagte Feststoff, der bevorzugt als Filterkuchen auf dem Trägermaterial bzw. als verdichtetes Präzipitat auf der Gefäßwandung belassen und jeweils in dieser Form direkt in Schritt c) weiter eingesetzt wird.

Das abgeschiedene Gemisch aus zumindest Nukleinsäure und Endotoxin wird gemäß Schritt c) des erfindungsgemäßen Verfahrens mit einer speziellen Waschlösung gewaschen. Dabei wird Endotoxin aus dem abgeschiedenen Gemisch größtenteils herausgewaschen, während überwiegend Nukleinsäure zurückbleibt.

Die in Schritt c) eingesetzte Waschlösung enthält zwingend eine spezielle organische Amin-Verbindung (vide supra).Die spezielle organische Amin-Verbindung wird beispielsweise unter Einhaltung vorgenannter zwingender Merkmale ausgewählt aus mindestens einer Verbindung der Gruppe, die gebildet wird aus Verbindungen mit einer Aminogruppe, Verbindungen mit mindestens zwei Aminogruppen, Verbindungen mit mindestens einer Aminogruppe und mindestens einer Hydroxylgruppe, Verbindungen mit mindestens zwei Aminogruppen und mindestens einer Hydroxylgruppe (besonders bevorzugt aus mindestens einer Verbindung der Gruppe, die gebildet wird aus Verbindungen mit einer Aminogruppe, Verbindungen mit mindestens einer Aminogruppe und mindestens einer Hydroxylgruppe).

Es ist erfindungsgemäß bevorzugt, wenn die besagte Amin-Verbindung eine Molmasse von 80 bis 500 g/mol besitzt.

Zur Verbesserung der Löslichkeit in Wasser ist es bevorzugt, wenn die Amin-Verbindung mindestens zwei Hydroxylgruppen aufweist.

Besonders geeignete organische Amin-Verbindungen weisen bevorzugt gegebenenfalls substituierte Kohlenwasserstoffreste mit 1 bis 6 Kohlenstoffatomen auf. Als Substituenten eignen sich insbesondere Hydroxy oder Alkoxy.

Die organische Amin-Verbindung der Waschlösung wird beispielsweise ausgewählt aus Triethylamin, Triethanolamin, 2-Amino-2-(hydroxymethyl)-propan-1,3-diol (TRIS), 2,2-Bis(hydroxymethyl)-2,2',2''-nitrilotriethanol (BIS-TRIS), 1,3-Bis[tris(hydroxymethyl)methylamino]propan (BIS-TRIS propan), Diisopropylamin, Triisopropylamin, oder Kombinationen davon.

Es ist erfindungsgemäß besonders bevorzugt, die organische Amin-Verbindung der Waschlösung im Schritt c) aus mindestens einer Verbindung der allgemeinen Formel (I) auszuwählen,

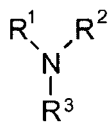
(I)

worin $R^1$, $R^2$ und $R^3$ unabhängig voneinander stehen für ein Wasserstoffatom, eine ($C_1$ bis $C_6$)-Alkylgruppe, eine ($C_1$ bis $C_6$)-Monohydroxyalkylgruppe, eine ($C_2$ bis $C_6$)-Dihydroxyalkylgruppe, eine ($C_3$ bis $C_6$)-Trihydroxyalkylgruppe,

mit der Maßgabe, dass das organische Amin der Formel (I) eine Molmasse von ≤ 500 g/mol aufweist, und mindestens einer der Reste $R^1$, $R^2$ und $R^3$ von einem Wasserstoffatom verschieden ist.

Besonders bevorzugt stehen $R^1$, $R^2$ und $R^3$ gemäß Formel (I) unabhängig voneinander für ein Wasserstoffatom, eine ($C_1$ bis $C_6$)-Alkylgruppe, eine ($C_2$ bis $C_6$)-Monohydroxyalkylgruppe, eine ($C_3$ bis $C_6$)-Dihydroxyalkylgruppe, eine ($C_4$ bis $C_6$)-Trihydroxyalkylgruppe,
Ganz besonders bevorzugt geeignete Amin-Verbindungen der Formel (I) weisen eine Molmasse von 80 bis 500 g/mol auf.

Ganz besonders bevorzugt wird die organische Amin-Verbindung der Waschlösung ausgewählt aus Triethylamin, Triethanolamin, 2-Amino-2-(hydroxymethyl)-propan-1,3-diol (TRIS), 2,2-Bis(hydroxymethyl)-2,2',2''-nitrilotriethanol (BIS-TRIS), 1,3-Bis[tris(hydroxymethyl)methylamino]propan (BIS-TRIS propan), oder Kombinationen davon.

Es stellten sich besonders gute Ergebnisse ein, wenn die Waschlösung gemäß Schritt c) die besagte organische Amin-Verbindung, insbesondere deren bevorzugte Vertreter, in einer Konzentration von 5 mmol/L bis 1000 mmol/L, bevorzugt von 200 mmol/L bis 800 mmol/L, weiter bevorzugt 200 mmol/L bis 700 mmol/L, besonders bevorzugt 250 mmol/L bis 650 mmol/L, enthält. Zur Berechnung der vorgenannten und auch nachfolgenden, bevorzugten Konzentration der Amin-Verbindung wird die zur Herstellung der Waschlösung eingesetzte Menge an Amin-Verbindung herangezogen.

Wenn in der erfindungsgemäßen Waschlösung geringe Mengen der besagten organischen Amin-Verbindung enthalten sind, i.e. von weniger als 100 mmol/L, so kann die erfindungsgemäße Abtrennung der Endotoxine bevorzugt durch Zusatz von mindestens einem chaotropen Salz (bevorzugte Vertretervide supra) verbessert werden. Dabei wird wiederum bevorzugt eine Gesamtmenge von 1 mol/L bis 6 mol/L, besonders bevorzugt 1 mol/L bis 4 mol/L, chaotropes Salz zugegeben. Dabei ist es wiederum bevorzugt, wenn eine erfindungsgemäße Waschlösung, die mindestens 100 mmol/L der besagten organischen Amin-Verbindung enthält, 0 bis 1 mol/L, insbesondere 0 bis 0,5 mol/L, ganz besonders bevorzugt 0 bis 0,1 mol/L zusätzliches chaotropes Salz enthält.

Die Waschlösung aus Schritt c) enthält zwingend mindestens ein von der besagten Amin-Verbindung verschiedenes, organisches Lösemittel. Das organische Lösemittel ist in der Waschlösung bevorzugt in einer Gesamtmenge von 20 bis 80 Gew.-% enthalten, bezogen auf das Gesamtgewicht der Waschlösung.

Es hat sich als vorteilhaft erwiesen, wenn die Waschlösung als bevorzugtes organisches Lösemittel mindestens einen Monoalkohol enthält. Besonders bevorzugt enthält die Waschlösung als organisches Lösemittel mindestens einen C1-C6-Monoalkohol, insbesondere Ethanol, Isopropanol oder Kombinationen davon. Dabei ist es wiederum ganz besonders bevorzugt, wenn die Waschlösung den C1-C6-Monoalkohol in einer Gesamtmenge von 20 Gew.-% bis 80 Gew.-% bezogen auf das Gesamtgewicht der Waschlösung enthält, insbesondere 30 Gew.-% bis 80 Gew.-%, bevorzugt 35 Gew.-% bis 80 Gew.-%, besonders bevorzugt 38 Gew.-% bis 80 Gew.-%.

Bei einer bevorzugten Ausführungsform ist der C1-C6-Monoalkohol Isopropanol und die Waschlösung enthält diesen in einer Gesamtmenge von 35 Gew.-% bis 80 Gew.-% bezogen auf das Gesamtgewicht der Waschlösung.

In einer hierzu alternativen bevorzugten Ausführungsform ist der C1-C6-Monoalkohol Ethanol und die Waschlösung enthält diesen in einer Gesamtmenge von 65 Gew.-% bis 80 Gew.-% bezogen auf das Gesamtgewicht der Waschlösung.

Diese Alkoholkonzentrationen sind bevorzugt, da hierdurch die abgeschiedenen Nukleinsäuren im abgeschiedenen Zustand verbleiben, also beim Waschen mit der Waschlösung nicht ungewollt in Lösung gehen. Auf diese Weise wird letztlich die Ausbeute an Nukleinsäuren erhöht.

Die Waschlösung gemäß Schritt c) weist zwingend einen pH-Wert (20 °C) im Bereich von pH 3,0 bis 8,5, bevorzugt von pH 4,0 bis pH 8,5, auf. Für optimierte Aufreinigungsergebnisse und eine verbesserte Nukleinsäureausbeute weist die besagte Waschlösung bevorzugt einen pH-Wert (20°C) im Bereich von pH 3,0 bis 7,5, von pH 3,0 bis 7,0, von pH 4,0 bis 7,5, von pH 4,0 bis 7,0, von pH 5,0 bis 7,5 oder besonders bevorzugt von pH 5,0 bis pH 7,0 auf.

In einer bevorzugten Ausführungsform der erfindungsgemäß eingesetzten Waschlösung, liegt das Stickstoffatom der Aminogruppe(n) der besagten Amin-Verbindung zu mindestens 50 %, besonders bevorzugt zu mindestens 60 %, ganz besonders bevorzugt zu mindestens 70 %, in protonierter Form vor. Zu diesem Zweck wird der pH-Wert der Waschlösung innerhalb des vorgegebenen pH-Wertbereiches bevorzugt so gewählt, dass das Stickstoffatom der Aminogruppe(n) der besagten Amin-Verbindung einen Protonierungsgrad von mindestens 50 %, besonders bevorzugt von mindestens 60 %, ganz besonders bevorzugt von mindestens 70 %, aufweist. Als Modellsystem zur Bereitstellung einer solchen bevorzugten Waschlösung wird eine wässrige Lösung der zur Herstellung beabsichtigten Einsatzmenge an Amin-Verbindung als Anfangskonzentration angenommen und der gemäß Henderson-Hasselbach-Gleichung errechnete pH-Wert (20°C) für den gewünschten Protonierungsgrad eingestellt:

$$pH = pKs + \log_{10}((1 - a) / a)$$

mit a = [Protonieurungsgrad der Amin - Verbindung in %] / 100Image

Die organischen Amin-Verbindungen mit besagtem Protonierungsgrad sind wiederum besonders bevorzugt in der zuvor als bevorzugt definierten Konzentration der Amin-Verbindung von 5 mmol/L bis 1000 mmol/l, insbesondere von 200 mmol/L bis 800 mmol/L, weiter bevorzugt 200 mmol/L bis 700 mmol/L, besonders bevorzugt 250 mmol/L bis 650 mmol/L, in der Waschlösung enthalten.

Für den Einsatz von zumindest TRIS als organische Amin-Verbindung hat es sich als optimal erwiesen, wenn die Waschlösung einen bevorzugten pH-Wert (20°C) im Bereich von pH 6,0 bis pH 8,5, insbesondere von pH 6,0 bis pH 7,0, aufweist. Dabei ist es wiederum besonders bevorzugt TRIS in der Waschlösung in einer Konzentration von mindestens 80 mmol/L, ganz besonders bevorzugt von mindestens 100 mmol/L, weiter bevorzugt mindestens 200 mmol/L, besonders bevorzugt mindestens 250, einzusetzen. Als Obergrenze der Gesamtkonzentration an besagter, organischer Amin-Verbindung kann für diese Ausführungsform 1000 mmol/L, insbesondere 800 mmol/L, weiter bevorzugt 700 mmol/L, besonders bevorzugt 650 mmol/L, vorteilhaft sein.

Für den Einsatz von BIS-TRIS als organische Amin-Verbindung hat es sich als optimal erwiesen, wenn die Waschlösung einen bevorzugten pH-Wert (20°C) im Bereich von pH 5,0 bis pH 8,0, insbesondere pH 5,5 bis pH 7,5, ganz besonders von pH 5,0 bis pH 7,0, aufweist. Dabei ist es wiederum besonders bevorzugt BIS-TRIS in der Waschlösung in einer Konzentration von mindestens 80 mmol/L, ganz besonders bevorzugt von mindestens 100 mmol/L, weiter bevorzugt mindestens 200 mmol/L, besonders bevorzugt mindestens 250, einzusetzen. Als Obergrenze der Gesamtkonzentration an besagter, organischer Amin-Verbindung kann für diese Ausführungsform 1000 mmol/L, insbesondere 800 mmol/L, weiter bevorzugt 700 mmol/L, besonders bevorzugt 650 mmol/L, vorteilhaft sein.

Dem Waschschritt c) des erfindungsgemäßen Verfahrens können gegebenenfalls weitere zusätzliche Waschschritte der in Schritt b) abgeschiedenen Bestandteile folgen oder vorangehen. Diese zusätzlichen Waschschritte der abgeschiedenen Nukleinsäure dienen bevorzugt der Entfernung von Kontaminanten, welche von Endotoxin verschieden sind.

Im Schritt d) des erfindungsgemäßen Verfahrens erfolgt das Inlösungbringen der verbliebenen Nukleinsäure aus den gemäß Schritt c) gewaschenen, abgeschiedenen Bestandteilen mittels eines Lösungspuffers und das Auffangen der gelösten Nukleinsäuren in einem separaten Auffang-Gefäß.

Beim Inlösungbringen wird die verbliebene, abgeschiedene Nukleinsäure in dem Lösungspuffer gelöst und von dem festen Trägermaterial entfernt. Bevorzugte Lösungspuffer, die sich für das Inlösungbringen der verbliebenen, abgeschiedenen Nukleinsäure eignen, weisen eine geringe lonenstärke auf. Beispielsweise eignet sich ein Lösungspuffer, der 5 bis 10 mM TRIS (gegebenenfalls in Kombination mit bis zu 1 mM Ethylendiamintetraessigsäure (EDTA)) enthält.

Das Inlösungbringen gemäß Schritt d) des erfindungsgemäßen Verfahrens erfolgt bevorzugt durch Umspülen des festen Trägermaterials mit dem Lösungspuffer und anschließendem Auffangen des Lösungspuffers in einem separaten Auffanggefäß. Das Umspülen wird bevorzugt durch einen Fluß des Lösungspuffers durch das feste Trägermaterial bewerkstelligt. Die Fließrichtung wird besonders bevorzugt durch Anlegen eines Vakuums oder durch Zentrifugation vorgegeben.

Die vorliegende Erfindung betrifft auch die Verwendung einer Waschlösung, der

(i) mindestens eine Amin-Verbindung mit mindestens zwei Kohlenstoffatomen und mit einer Molmasse von ≤ 500

g/mol und

(ii) mindestens ein von besagter Amin-Verbindung verschiedenes organisches Lösemittel, enthält und

einen pH-Wert (20 °C) im Bereich von pH 3,0 bis pH 8,5 aufweist,
zur wenigstens teilweisen Entfernung von Endotoxinen aus einer Nukleinsäure-haltigen Probe, wobei die Waschlösung dadurch gekennzeichnet ist, dass die Waschlösung die besagte organische Amin-Verbindung in einer Konzentration von 200 mmol/L bis 1000 mmol/L, enthält und im Rahmen der wenigstens teilweisen Entfernung jeweils bezogen auf die Gesamtmenge an Endotoxin in der flüssigen Probe wenigstens 50 Gew.-% Endotoxin entfernt werden.

Als bevorzugte Nukleinsäure können in der Probe die in den Ausführungsformen des ersten Erfindungsgegenstandes beschriebenen Nukleinsäuren (insbesondere Plasmid DNA) enthalten sein.

Die Waschlösung ist im Sinne der erfindungsgemäßen Verwendung bevorzugt geeignet, wenn die Nukleinsäure und das Endotoxin auf einem festen Trägermaterial gebunden, insbesondere adsorbiert, vorliegen.

Die Erfindung betrifft ein Kit zur Aufreinigung von Nukleinsäuren, insbesondere Plasmid DNA, umfassend eine Waschlösung, der

(i) mindestens eine Amin-Verbindung mit mindestens zwei Kohlenstoffatomen und mit einer Molmasse von ≤ 500 g/mol und
(ii) mindestens ein von besagter Amin-Verbindung verschiedenes organisches Lösemittel, enthält und

einen pH-Wert (20 °C) im Bereich von pH 3,0 bis pH 8,5 aufweist
sowie mindestens einen zusätzlichen Bestandteil, ausgewählt aus einer Bedienungsanleitung zur Durchführung eines Verfahrens des ersten Erfindungsgegenstandes, Bindepuffer und Lösungspuffer zum Inlösungbringen der wenigstens teilweise von Endotoxinen gereinigten Nukleinsäuren, wobei die Waschlösung dadurch gekennzeichnet ist, dass die Waschlösung die besagte organische Amin-Verbindung in einer Konzentration von 200 mmol/L bis 1000 mmol/L enthält und im Rahmen der wenigstens teilweisen Entfernung jeweils bezogen auf die Gesamtmenge an Endotoxin in der flüssigen Probe wenigstens 50 Gew.-% Endotoxin entfernt werden.

Alle bevorzugten Ausführungsformen des Bindepuffers des ersten Erfindungsgegenstandes gelten ebenso für das Kits des dritten Erfindungsgegenstandes.

Alle bevorzugten Ausführungsformen des Lösungspuffers des ersten Erfindungsgegenstandes gelten ebenso für das Kit des dritten Erfindungsgegenstandes.

Die Erfindung betrifft insbesondere die folgenden Ausführungsformen:
Nach einer ersten Ausführungsform betrifft die Erfindung ein Verfahren zur Aufreinigung von Nukleinsäuren, insbesondere von Plasmid DNA, aus einer Nukleinsäure-haltigen, biologischen Probe, umfassend die folgenden Schritte:

a) Bereitstellen einer flüssigen Probe, enthaltend Nukleinsäure und Endotoxin jeweils in gelöster Form;

b) Abscheidung zumindest von Nukleinsäure und Endotoxin aus der flüssigen Probe heraus;

c) Waschen der im Schritt b) abgeschiedenen Bestandteile der flüssigen Probe zum wenigstens teilweisen Entfernen des Endotoxins mit mindestens einer Waschlösung, die

(i) mindestens eine Amin-Verbindung mit mindestens zwei Kohlenstoffatomen und mit einer Molmasse von ≤ 500 g/mol und;
(ii) mindestens ein von besagter Amin-Verbindung verschiedenes organisches Lösemittel, enthält und einen pH-Wert bei 20°C im Bereich von pH 3,0 bis pH 8,5 aufweist, und;

d) Inlösungbringen der verbliebenen Nukleinsäure aus den gemäß Schritt c) gewaschenen, abgeschiedenen Bestandteilen mittels eines Lösungspuffers und Auffangen der gelösten Nukleinsäuren in einem separaten Auffang-Gefäß.

Nach einer zweiten Ausführungsform betrifft die Erfindung Verfahren nach Ausführungsform 1, dadurch gekennzeichnet, dass im Schritt b) zumindest Nukleinsäure und Endotoxin durch Bindung, insbesondere durch Adsorption oder Fällung, an die Oberfläche eines festen Trägermaterials abgeschieden werden.

Nach einer dritten Ausführungsform betrifft die Erfindung Verfahren nach Ausführungsform 2, dadurch gekennzeichnet, dass die Probe im Schritt b) zur Abscheidung an das Trägermaterial mit einem Bindepuffer versetzt wird.

Nach einer vierten Ausführungsform betrifft die Erfindung Verfahren nach Ausführungsform 1, dadurch gekennzeichnet, dass im Schritt b) zumindest die Nukleinsäure und das Endotoxin als Feststoff durch Fällung abgeschieden

werden.

Nach einer fünften Ausführungsform betrifft die Erfindung Verfahren nach Ausführungsform 4, dadurch gekennzeichnet, dass die gefällte Nukleinsäure und das Endotoxin als Feststoff

(i) durch Filtration auf der Oberfläche eines festen Trägermaterials gesammelt, oder
(ii) durch Zentrifugation verdichtet,

und jeweils von der Mutterlösung abgetrennt werden.

Nach einer sechsten Ausführungsform betrifft die Erfindung Verfahren nach einer der Ausführungsformen 2, 3 oder 5, dadurch gekennzeichnet, dass das Trägermaterial ausgewählt wird aus mineralischen Trägermaterialien, insbesondere Quarzfasern, Silica, Glas, Aluminiumoxid, Zeolith, Titandioxid, Zirkondioxid oder Kombinationen daraus.

Nach einer siebten Ausführungsform betrifft die Erfindung Verfahren nach einer der vorangehenden Ausführungsformen, dadurch gekennzeichnet, dass die organische Amin-Verbindung der Waschlösung im Schritt c) ausgewählt wird aus mindestens einer Verbindung der allgemeinen Formel (I),

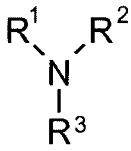
(I)

worin $R^1$, $R^2$ und $R^3$ unabhängig voneinander stehen für ein Wasserstoffatom, eine ($C_1$ bis $C_6$)-Alkylgruppe, eine ($C_1$ bis $C_6$)-Monohydroxyalkylgruppe, eine ($C_2$ bis $C_6$)-Dihydroxyalkylgruppe, eine ($C_3$ bis $C_6$)-Trihydroxyalkylgruppe, mit der Maßgabe, dass das organische Amin der Formel (I) eine Molmasse von ≤ 500 g/mol aufweist, und mindestens einer der Reste $R^1$, $R^2$ und $R^3$ von einem Wasserstoffatom verschieden ist.

Nach einer achten Ausführungsform betrifft die Erfindung Verfahren nach einer der vorangehenden Ausführungsformen, dadurch gekennzeichnet, dass die organische Amin-Verbindung der Waschlösung ausgewählt wird, aus Triethylamin, Triethanolamin, 2-Amino-2-(hydroxymethyl)-propan-1,3-diol (TRIS), 2,2-Bis(hydroxy-methy))-2,2',2"-nitrilotriethanol (BIS-TRIS), 1,3-Bis[tris(hydroxymethyl)methylamino]propan (BIS-TRIS propan), oder Kombinationen davon.

Nach einer neunten Ausführungsform betrifft die Erfindung Verfahren nach einer der vorangehenden Ausführungsformen, dadurch gekennzeichnet, dass die Waschlösung gemäß Schritt c) die besagte organische Amin-Verbindung in einer Konzentration von 5 mmol/L bis 1000 mmol/L, insbesondere von 200 mmol/L bis 800 mmol/L, enthält.

Nach einer zehnten Ausführungsform betrifft die Erfindung Verfahren nach einer der vorangehenden Ausführungsformen, dadurch gekennzeichnet, dass das Stickstoffatom der Aminogruppe(n) der besagten Amin-Verbindung zu mindestens 50 %, bevorzugt zu mindestens 60 %, besonders bevorzugt zu mindestens 70 %, in protonierter Form vorliegt.

Nach einer elften Ausführungsform betrifft die Erfindung Verfahren nach einer der vorangehenden Ausführungsformen, dadurch gekennzeichnet, dass die Waschlösung gemäß Schritt c) als Lösemittel mindestens einen C1-C6-Monoalkohol, insbesondere Ethanol, Isopropanol oder Kombinationen davon, enthält.

Nach einer elften Ausführungsform betrifft die Erfindung Verfahren nach Ausführungsform 11, dadurch gekennzeichnet, dass die Waschlösung den C1-C6-Monoalkohol in einer Gesamtmenge von 20 Gew.-% bis 80 Gew.-% bezogen auf das Gesamtgewicht der Waschlösung enthält.

Nach einer dreizehnten Ausführungsform betrifft die Erfindung Verfahren nach einer der vorangehenden Ausführungsformen, dadurch gekennzeichnet, dass die Waschlösung gemäß Schritt c) einen pH-Wert (20 °C) im Bereich von pH 5,0 bis pH 7,0 aufweist.

Nach einer vierzehnten Ausführungsform betrifft die Erfindung Verfahren nach einer der vorangehenden Ausführungsformen, dadurch gekennzeichnet, dass im Rahmen der wenigstens teilweisen Entfernung jeweils bezogen auf die Gesamtmenge an Endotoxin in der flüssigen Probe wenigstens 50 Gew.-% Endotoxin, bevorzugt wenigstens 60 Gew.-% Endotoxin, besonders bevorzugt wenigstens 70 Gew.-% Endotoxin, entfernt werden.

Nach einer fünfzehnten Ausführungsform betrifft die Erfindung die Verwendung einer Waschlösung, die

(i) mindestens eine Amin-Verbindung mit mindestens zwei Kohlenstoffatomen und mit einer Molmasse von ≤ 500 g/mol und

(ii) mindestens ein von besagter Amin-Verbindung verschiedenes organisches Lösemittel, enthält und

einen pH-Wert (20 °C) im Bereich von pH 3,0 bis pH 8,5 aufweist,

zur wenigstens teilweisen Entfernung von Endotoxinen aus einer Nukleinsäure-haltigen Probe, die dadurch gekennzeichnet ist, dass die gefällte Nukleinsäure und das Endotoxin als Feststoff

i) durch Filtration auf der Oberfläche eines festen Trägermaterials gesammelt, oder

ii) durch Zentrifugation verdichtet,

und jeweils von der Mutterlösung abgetrennt werden.

Nach einer sechzehnten Ausführungsform betrifft die Erfindung eine Verwendung nach Ausführungsform 15, dadurch gekennzeichnet, dass die Nukleinsäure und das Endotoxin auf einem festen Trägermaterial gebunden, insbesondere adsorbiert, vorliegen.

Nach einer siebzehnten Ausführungsform betrifft die Erfindung ein Kit zur Aufreinigung von Nukleinsäuren, insbesondere Plasmid DNA, umfassend eine Waschlösung, die

(i) mindestens eine Amin-Verbindung mit mindestens zwei Kohlenstoffatomen und mit einer Molmasse von $\leq$ 500 g/mol und

(ii) mindestens ein von besagter Amin-Verbindung verschiedenes organisches Lösemittel, enthält und

einen pH-Wert (20 °C) im Bereich von pH 3,0 bis pH 8,5 aufweist
sowie mindestens einen zusätzlichen Bestandteil, ausgewählt aus einer Bedienungsanleitung zur Durchführung eines Verfahrens nach einer der Ausführungsformen 1 bis 14, einem Bindepuffer und einem Lösungspuffer zum Inlösungbringen der wenigstens teilweise von Endotoxinen gereinigten Nukleinsäuren das dadurch gekennzeichnet ist, dass die Waschlösung die besagte organische Amin-Verbindung in einer Konzentration von 200 mmol/L bis 1000 mmol/L enthält, und
im Rahmen der wenigstens teilweisen Entfernung jeweils bezogen auf die Gesamtmenge an Endotoxin in der flüssigen Probe wenigstens 50 Gew.-% Endotoxin entfernt werden.

Beispiele

Beispiel 1:

Eine Übernachtkultur von E. coli DH5α™, transformiert mit dem Plasmid pcDNA™3.1(+) wurde in einem ampicillinhaltigen Nährmedium bereitgestellt. Unterschiedlich große Aliquots der Kultur ä 1 ml, 2 ml und 3 ml wurden bei 10'000 x g für 5 min abzentrifugiert, der Überstand wurde verworfen. Die Pellets wurden in einem Resuspensionspuffer enthaltend EDTA, Tris und RNase A resuspendiert, mit einem Lysepuffer enthaltend NaOH und SDS lysiert und durch einen Neutralisationspuffer enthaltend Kaliumacetat neutralisiert. Die Lysate wurden durch Zentrifugation bei 10'000 x g für 10 min geklärt und der klare Überstand mit einer Guanidiniumhydrochloridlösung gemischt. Das Gemisch wurde in Silicaschichten enthaltende Spinsäulen gefüllt und durch Zentrifugation bei 10'000 x g für 1 min durch das Silica-Trägermaterial in ein Auffanggefäß gespült. In Folge wurde für jedes pelletierte Bakterienkulturvolumen eine Waschlösung in die Spinsäulen gefüllt, wobei es folgende Variation gab:

- keine Waschlösung
- 30% Isopropanol, 7 mM TRIS, 4.55 M Guanidiniumhydrochlorid, pH 7.5
- 50% Isopropanol, 200 mM BIS-TRIS, pH 5.5

Die Säulen wurden bei 10'000 x g für 1 min zentrifugiert, wobei die Waschlösung durch das Silica-Trägermaterial in ein Auffanggefäß gespült wurde. Dieser Vorgang wurde einmal wiederholt. Anschließend wurde ein Waschschritt mit einer bekannten Waschlösung enthaltend 80% Ethanol durchgeführt. Auch hier wurde zum Durchspülen des Silica-Trägermaterials bei 10'000 x g für 1 min zentrifugiert. Zum Trocknen wurden die Säulen leer bei 10'000 x g für 1 min zentrifugiert. Eluiert wurde die DNA nach Zusatz von 100 μl Lösungspuffer (Wasser mit 5 mM Tris-Cl, pH 8.5) ebenfalls durch Zentrifugation bei 10'000 x g für 1 min. Die Ausbeute wurde mit einem Photometer durch Messung der Extinktion bei 260 nm bestimmt, der Endotoxingehalt durch Messung mittels pyrochromem LAL-Test (Cape Cod).

Die folgende Tabelle zeigt die Messergebnisse:

| Waschlösung | µg DNA/ Eluat | relative Ausbeute/ % | EU/ µg | EndotoxinEntfernung/ % |
|---|---|---|---|---|
| **1 ml Bakterienkultur** | | | | |
| ohne speziellen Waschschritt | 12,9 | 100% | 125 | 0 |
| 30% Isopropanol, 7 mM TRIS, 4.55 M Guanidiniumhydrochlorid, pH 7.5 | 13,5 | 105% | 15 | 88 |
| 50% Isopropanol + 200 mM BIS-TRIS, pH 5.5 | 12,1 | 94% | 14 | 89 |
| **2 ml Bakterienkultur** | | | | |
| ohne speziellen Waschschritt | 16,9 | 100% | 632 | 0 |
| 30% Isopropanol, 7 mM TRIS, 4.55 M Guanidiniumhydrochlorid, pH 7.5 | 15,4 | 91% | 102 | 84 |
| 50% Isopropanol + 200 mM BIS-TRIS, pH 5.5 | 15,0 | 89% | 14 | 98 |
| **3 ml Bakterienkultur** | | | | |
| ohne speziellen Waschschritt | 18,8 | 100% | 914 | 0 |
| 30% Isopropanol, 7 mM TRIS, 4.55 M Guanidiniumhydrochlorid, pH 7.5 | 17,1 | 91% | 128 | 86 |
| 50% Isopropanol + 200 mM BIS-TRIS, pH 5.5 | 16,7 | 89% | 12 | 99 |

Bereits die Anwesenheit kleiner Mengen TRIS reichte aus, um die Endotoxinkonzentration in den Eluaten deutlich zu senken. Ein signifikanter Einfluss auf die DNA-Ausbeute war nicht zu erkennen.

Beispiel 2:

Eine Übernachtkultur von E. coli DH5 α ™, transformiert mit dem Plasmid pcDNA™3.1(+) wurde in einem ampicillinhaltigen Nährmedium bereitgestellt. Aliquots der Kultur ä 25 ml wurden bei 4’500 x g für 10 min abzentrifugiert, der Überstand wurde verworfen. Die Pellets wurden in einem Resuspensionspuffer enthaltend EDTA, Tris und RNase A resuspendiert, mit einem Lysepuffer, enthaltend NaOH und SDS, lysiert und durch einen Neutralisationspuffer, enthaltend Kaliumacetat, neutralisiert. Die Lysate wurden durch Zentrifugation über eine Filtersäule enthaltend Polyethylenfritten und Silica bei 3’000 x g für 2 min geklärt und der klare Durchfluss mit einer Fällungslösung enthaltend 85% Tripropylenglycol und 2.5% Ethanol gemischt. Das Gemisch wurde in Silicaschichten enthaltende Säulen gefüllt und durch Anlegen eines Unterdrucks von -0,3 bar durch das Silica-Trägermaterial in ein Auffanggefäß gezogen. In Folge wurde zunächst mit Waschlösungen enthaltend 200 mM oder 400 mM TRIS oder BIS-TRIS bei pH 7 oder pH 8 für TRIS und pH 5.5 oder pH 6.5 für BIS-TRIS sowie zur Kontrolle ohne Waschschritt und mit 50% Isopropanol ohne weitere Zusätze gewaschen und anschließend mit einer bekannten Waschlösung enthaltend 80% Ethanol gespült. Auch hier wurde zum Durchspülen der Silica ein Unterdruck von -0.3 bar verwendet. Zum Trocknen wurden die Säulen leer bei 10’000 x g für 1 min zentrifugiert. Eluiert wurde die DNA nach Zusatz von 200 µl Lösungspuffer (Wasser mit 5 mM Tris-Cl, pH 8.5) ebenfalls durch Zentrifugation bei 10’000 x g für 1 min. Die Ausbeute wurde mit einem Photometer durch Messung der Extinktion bei 260 nm bestimmt, der Endotoxingehalt durch Messung mittels pyrochromem LAL-Test (Cape Cod).

Die folgende Tabelle zeigt die Messergebnisse:

| Waschlösung | µg DNA/Eluat | EU/µg | Endotoxin-Entfernung/% |
|---|---|---|---|
| **TRIS pH 7.0** | | | |
| 200 mM | 79 | 25 | 80 |
| 400mM | 73 | 13 | 89 |
| **TRIS pH 8.0** | | | |
| 200 mM | 73 | 18 | 86 |
| 400 mM | 78 | 17 | 86 |
| **BIS-TRIS pH 5.5** | | | |
| 200 mM | 73 | 12 | 90 |
| 400 mM | 74 | 18 | 85 |

(fortgesetzt)

| Waschlösung | µg DNA/Eluat | EU/µg | Endotoxin-Entfernung/% |
|---|---|---|---|
| **BIS-TRIS pH 6.5** | | | |
| 200 mM | 70 | 5 | 96 |
| 400 mM | 66 | 6 | 94 |
| ohne Waschschritt | 89 | 123 | 0 |
| 50% Isopropanol | 13 | | |

Die nicht erfindungsgemäße Waschlösung aus einer Lösung von 50% Isopropanol war alleine nicht dazu in der Lage, die DNA auf der Säule zu halten.

Beispiel 3:

Eine Übernachtkultur von E. coli DH5α™, transformiert mit dem Plasmid pcDNA™3.1(+) wurde in einem ampicillinhaltigen Nährmedium bereitgestellt. Aliquots der Kultur ä 50 ml wurden bei 4'500 x g für 10 min abzentrifugiert, der Überstand wurde verworfen. Die Pellets wurden in einem Resuspensionspuffer, enthaltend EDTA, Tris und RNase A, resuspendiert, mit einem Lysepuffer, enthaltend NaOH und SDS, lysiert und durch einen Neutralisationspuffer, enthaltend Kaliumacetat, neutralisiert. Die Lysate wurden durch Zentrifugation über eine Filtersäule, enthaltend Polyethylenfritten und Silica bei 3'000 x g für 2 min geklärt und der klare Durchfluss mit einer Fällungslösung enthaltend 85% Tripropylenglycol und 2.5% Ethanol gemischt. Das Gemisch wurde in Silicaschichten enthaltende Säulen gefüllt und durch Anlegen eines Unterdrucks von -0,3 bar durch die Silica in ein Auffanggefäß gezogen. In Folge wurden Waschlösungen mit der folgenden Zusammensetzung in die Säulen gefüllt und durch Anlegen eines Unterdrucks von -0,3 bar durch die Silica in ein Auffanggefäß gezogen:

- ohne Waschschritt
- 50% Isopropanol + 200 mM BIS-TRIS, pH 6.0 (erfindungsgemäß)
- 50% Isopropanol + 100 mM NaCl
- 50% Isopropanol + 500 mM NaCl
- 50% Isopropanol + 1 M NaCl
- 50% Isopropanol + 1 M Guanidin HCl
- 50% Isopropanol + 2 M Guanidin HCl
- 50% Isopropanol + 3 M Guanidin HCl
- 50% Isopropanol + 100 mM Natriumacetat, pH 5,0
- 50% Isopropanol + 250 mM Natriumacetat, pH 5,0
- 50% Isopropanol + 500 mM Natriumacetat, pH 5,0

Anschließend wurden die Säulen mit einer bekannten Waschlösung enthaltend 80% Ethanol gespült. Auch hier wurde zum Durchspülen des Silica-Trägermaterials ein Unterdruck von -0.3 bar verwendet. Zum Trocknen wurden die Säulen leer bei 10'000 x g für 1 min zentrifugiert. Eluiert wurde die DNA nach Zusatz von 200 µl Lösungspuffer (Wasser mit 5 mM Tris-Cl, pH 8.5) ebenfalls durch Zentrifugation bei 10'000 x g für 1 min. Die Ausbeute wurde mit einem Photometer durch Messung der Extinktion bei 260 nm bestimmt, der Endotoxingehalt durch Messung mittels pyrochromem LAL-Test (Cape Cod).

Folgende Messergebnisse wurden erhalten:

| Waschlösung | µg DNA/ Eluat | relative Ausbeu te/% | EU/µg | Endotoxin Entfernung/% |
|---|---|---|---|---|
| ohne Waschschritt | 635 | 100% | 473 | 0 |
| 50% Isopropanol + 200 mM BIS-TRIS, pH 6.0 | 597 | 94% | 34 | 93 |
| 50% Isopropanol + 100 mM Natriumchlorid | 625 | 98% | 448 | 5 |
| 50% Isopropanol + 500 mM Natriumchlorid | 603 | 95% | 511 | 0 |
| 50% Isopropanol + 1000 mM Natriumchlorid | 617 | 97% | 305 | 36 |
| 50% Isopropanol + 1 M Guanidin HCl | 617 | 97% | 317 | 33 |
| 50% Isopropanol + 2 M Guanidin HCl | 612 | 96% | 464 | 2 |
| 50% Isopropanol + 3 M Guanidin HCl | 586 | 92% | 154 | 67 |

(fortgesetzt)

| Waschlösung | μg DNA/ Eluat | relative Ausbeu te/% | EU/μg | Endotoxin Entfernung/% |
|---|---|---|---|---|
| 50% Isopropanol + 100 mM Natriumacetat, pH 5.0 | 641 | 101% | 369 | 22 |
| 50% Isopropanol + 250 mM Natriumacetat, pH 5.0 | 644 | 101% | 388 | 18 |
| 50% Isopropanol + 500 mM Natriumacetat, pH 5.0 | 606 | 95% | 275 | 42 |

Deutlich zu erkennen ist, dass in allen Fällen die Salzkonzentration ausreichend war, um die DNA während des Waschschrittes auf der Säule zu halten. Eine signifikante Entfernung von Endotoxinen gelang jedoch weder mit Salz beziehungsweise Isopropanol alleine noch über einen pH-Wert Effekt. Auch Acetat kann die Endotoxine nur unvollständig abtrennen.

**Vergleichsversuche gegenüber WO 2005/111059 A2**

Die folgenden Vergleichsversuche zeigen eine Gegenüberstellung der Erfindung gegenüber dem Stand der Technik in Form der WO 2005/111059 A2. Zunächst wurden zwei Waschlösungen gemäß der WO 2005/111059 A2 erzeugt:

Waschlösung 1:

| | |
|---|---|
| 100 mM Tris | 1.211 g/ 100 mL |
| 4.5 M Guanidinhydrochlorid | 43.0 g/ 100 mL |
| Lösen in H2O | |
| Anpassung auf pH 6.9 mit Essigsäure | |
| 25% Isopropanol | 25 mL/ 100 mL |
| ad 100 mL mit H2O | |

Waschlösung 2:

| | |
|---|---|
| 10 mM Tris | 0,121 g/ 100 mL |
| 10 mM NaCl | 0,058 g/ 100 mL |
| Lösen in H2O | |
| Anpassung auf pH 8.0 mit HCl | |
| 80% Ethanol | 80 mL/ 100 mL |
| ad 100 mL mit H2O | |

Für die folgenden Versuche wurden weiterhin noch die folgenden Chemikalien eingesetzt:

| | |
|---|---|
| Resuspensionspuffer: | 50 mM Tris-Cl, 10 mM EDTA, pH 8.0, 400 μg/mL RNase A |
| Lysepuffer: | 1% SDS, 200 mM NaOH |
| Neutralisierungspuffer: | 2.8 M Kaliumacetat, pH 5.1 |
| Bindungspuffer: | 8 M Guanidinhydrochlorid |
| Fällungslösung: | 85% Tripropylenglycol, 2.5% Ethanol |
| Endotoxin Entfernungspuffer: | 8% Triethanolamin, 50% Isopropanol, pH 6.0 |
| Alkoholischer Waschpuffer: | 2.5 mM Tris, 20 mM NaCl, 80% Ethanol, pH 7.5 |

**Vergleichsversuch 1**

300 mL LB-Medium und 100 μg/mL Ampicillin wurden mit E. coli Top10, enthaltend das Plasmid pcDNATM3.1 (+), inokkuliert und unter Schütteln bei 37 °C über Nacht inkubiert. Nach 16 Stunden Inkubation wurde eine OD600 von 4.6 gemessen. Die Kultur wurde in sechs Aliquots ä 50 mL aufgeteilt, die durch Zentrifugation bei 6000 x g für 5 min in

Bakterienpellet und Überstand getrennt wurden.

Die Überstände wurden verworfen und die Bakterienpellets in jeweils 5 mL Resuspensionspuffer aufgenommen. Die Lyse erfolgte durch Zugabe von je 5 mL Lysepuffer und Inkubation bei Raumtemperatur für 2 Minuten. Durch Zugabe von je 5 mL Neutralisierungspuffer wurde ein Präzipitat erzeugt, welches durch Zentrifugation über eine Filtersäule, enthaltend PE-Filterfritten und Silicapapier, bei 3000 x g für 2 min abgetrennt wurde. Die klaren Überstände mit den freigesetzten Plasmiden wurden gepoolt, durch Vortexen gemischt und zu 6x 12 mL neu aliquotiert.

Zu jeweils 12 mL Masterlysat wurden 3 mL Bindepuffer gegeben und durch Vortexen vermischt. Die Chaotropsalzkonzentration entspricht mit effektiv 1.6 M Guanidinhydrochlorid der in D1 gelehrten Konzentration.

Die Ansätze wurden auf mit drei Lagen Silicapapier gefüllte Säulen geladen (Durchmesser der Filter: 7.25 mm) und sollten durch Anlegen von Vakuum (900 mbar Druckreduktion zum Normaldruck) durch die Silicamembran gezogen werden. Bereits nach wenigen Millilitern verstopften die Säulen jedoch komplett.

**Vergleichsversuch 2**

300 mL LB-Medium plus 100 μg/mL Ampicillin wurden mit E. coli Top10, enthaltend das Plasmid pcDNATM3.1 (+), inokkuliert und unter Schütteln bei 37 °C über Nacht inkubiert. Nach 16 Stunden Inkubation wurde eine OD600 von 4.6 gemessen. Die Kultur wurde in sechs Aliquots ä 50 mL aufgeteilt, die durch Zentrifugation bei 6000 x g für 5 min in Bakterienpellet und Überstand getrennt wurden.

Die Überstände wurden verworfen und die Bakterienpellets in jeweils 5 mL Resuspensionspuffer aufgenommen. Die Lyse erfolgte durch Zugabe von je 5 mL Lysepuffer und Inkubation bei Raumtemperatur für 2 Minuten. Durch Zugabe von je 5 mL Neutralisierungspuffer wurde ein Präzipitat erzeugt, welches durch Zentrifugation über eine Filtersäule, enthaltend PE-Filterfritten und Silicapapier, bei 3000 x g für 2 min abgetrennt wurde. Die klaren Überstände mit den freigesetzten Plasmiden wurden gepoolt, durch Vortexen gemischt und zu 6x 12 mL neu aliquotiert. Zu jeweils 12 mL Masterlysat wurden 6 mL Fällungslösung gegeben und durch Vortexen vermischt.

Die Ansätze wurden auf mit drei Lagen Silicapapier gefüllte Säulen geladen (Durchmesser der Filter: 7.25 mm) und durch Anlegen von Vakuum (300 mbar Druckreduktion zum Normaldruck) durch die Silica gezogen. Nach vollständigem Durchlauf der Lösung wurde in Dreifachbestimmung zunächst mit je 2 mL Waschlösung 1 gemäß WO 2005/111059 A2 und anschließend mit je 4 mL Waschlösung 2 gemäß WO 2005/111059 A2 gewaschen. Alternativ wurde in Dreifachbestimmung mit je 2 mL Endotoxin Entfernungspuffer und anschließend mit je 4 mL alkoholischem Waschpuffer gewaschen. Die Waschschritte erfolgten immer bei einer Druckreduktion von 0.3 bar im Vergleich zum Normaldruck. Alle sechs Ansätze wurden durch Zentrifugation bei 11'000 x g für 1 min von Ethanolresten befreit. Eluiert wurde durch Zugabe von je 500 μL H2O und Zentrifugation bei 11 '000 x g für 1 min.

Die eluierte DNA wurde photometrisch vermessen (Spektrum von 200 nm bis 300 nm) und der Endotoxingehalt mittels chromogenem LAL-Test (Pyrochrome, 260260PA-25, Pyroquant Diagnostik GmbH) gemessen. Als Standard für die Quantifizierung wurde ein Kontroll-Standard Endotoxine (EC010-5, Pyroquant Diagnostik GmbH) verwendet. Für die Messung wurden die Eluate 1:1000 mit H2O verdünnt.

**Ergebnis Vergleichsversuch 1**

Dieses Beispiel zeigt, dass die aus WO 2005/111059 A2 bekannten Waschlösungen spezielle Rahmenbedingungen erfordern, zum Beispiel eine breitere Filterfläche oder die Anwesenheit von Detergenz im Bindepuffer. Diese Erfordernisse führen zu einem erhöhten Aufwand, der gerade bei Reihenuntersuchungen als nachteilig empfunden werden kann.

**Ergebnis Vergleichsversuch 2**

Die photometrische Messung gemäß Fig. 1 lieferte folgende Ergebnisse:

| Erf. | Vgl. | Erf. | Vgl. | Erf. | Vgl. |
|---|---|---|---|---|---|
| μg DNA/ Eluat | | A260/A280 | | A260/A230 | |
| 454 | 12 | 1,86 | 1,76 | 2,28 | 1,89 |
| 452 | 12 | 1,86 | 1,78 | 2,28 | 1,93 |
| 431 | 9 | 1,86 | 1,77 | 2,29 | 1,89 |
| 446 | 11 | 1,86 | 1,77 | 2,28 | 1,90 |

In Fig. 1 sind die erfindungsgemäßen Ergebnisse in der durchgehenden, diejenigen der Vorgehensweise gemäß WO 2005/111059 A2 gestrichelt dargestellt. Scheinbar wurde durch Waschpuffer 1 und Waschpuffer 2 aus WO 2005/111059 A2 ein Großteil der DNA entfernt. Die Reinheitsverhältnisse zeigen in allen Fällen saubere DNA an (Soll: A260/A280 = 1.8 bis 1.9, A260/A230 = 1.9 bis 2.3).

LAL-Test:

| | EU/Eluat | μg DNA/Eluat | EU/μg |
|---|---|---|---|
| Erf. | 4796 | 454 | 11 |
| | 3965 | 452 | 9 |
| | 3178 | 431 | 7 |
| | 3980 | 446 | 9 |
| Vgl. | 9840 | 12 | 795 |
| | 10207 | 12 | 823 |
| | 10259 | 9 | 1207 |
| | 10102 | 11 | 911 |

Obwohl die DNA-Menge bei WO 2005/111059 A2 deutlich geringer ist (ca. 2.5% im Vergleich zur Erfindung), ist der Gehalt an Endotoxinen etwa 250% höher. Es konnte mit der erfindungsgemäßen Lösung folglich ein Weg gefunden werden, DNA und Endotoxine abzuscheiden, die abgeschiedenen Endotoxine dann jedoch wieder durch die erfindungsgemäße Lösung zu entfernen.

## Patentansprüche

**1.** Verfahren zur Aufreinigung von Nukleinsäuren, insbesondere von Plasmid DNA, aus einer Nukleinsäure-haltigen, biologischen Probe, umfassend die folgenden Schritte:

a) Bereitstellen einer flüssigen Probe, enthaltend Nukleinsäure und Endotoxin jeweils in gelöster Form;
b) Abscheidung zumindest von Nukleinsäure und Endotoxin aus der flüssigen Probe heraus;
c) Waschen der im Schritt b) abgeschiedenen Bestandteile der flüssigen Probe zum wenigstens teilweisen Entfernen des Endotoxins mit mindestens einer Waschlösung, die

(i) mindestens eine Amin-Verbindung mit mindestens zwei Kohlenstoffatomen und mit einer Molmasse von ≤ 500 g/mol und;
(ii) mindestens ein von besagter Amin-Verbindung verschiedenes organisches Lösemittel, enthält und

einen pH-Wert im Bereich von pH 3,0 bis pH 8,5 aufweist, und;
d) Inlösungbringen der verbliebenen Nukleinsäure aus den gemäß Schritt c) gewaschenen, abgeschiedenen Bestandteilen mittels eines Lösungspuffers und Auffangen der gelösten Nukleinsäuren in einem separaten Auffang-Gefäß,

**dadurch gekennzeichnet, dass**
die Waschlösung gemäß Schritt c) die besagte organische Amin-Verbindung in einer Konzentration von 200 mmol/L bis 1000 mmol/L enthält, und
im Rahmen der wenigstens teilweisen Entfernung, jeweils bezogen auf die Gesamtmenge an Endotoxin in der flüssigen Probe, wenigstens 50 Gew.-% Endotoxin entfernt werden.

**2.** Verfahren nach Anspruch 1, **dadurch gekennzeichnet, dass** im Schritt b) zumindest Nukleinsäure und Endotoxin durch Bindung, insbesondere durch Adsorption oder Fällung, an die Oberfläche eines festen Trägermaterials abgeschieden werden.

3. Verfahren nach Anspruch 2, **dadurch gekennzeichnet, dass** die Probe im Schritt b) zur Abscheidung an das Trägermaterial mit einem Bindepuffer versetzt wird.

4. Verfahren nach Anspruch 1, **dadurch gekennzeichnet, dass** im Schritt b) zumindest die Nukleinsäure und das Endotoxin als Feststoff durch Fällung abgeschieden werden.

5. Verfahren nach Anspruch 4, **dadurch gekennzeichnet, dass** die gefällte Nukleinsäure und das Endotoxin als Feststoff

   i) durch Filtration auf der Oberfläche eines festen Trägermaterials gesammelt, oder
   ii) durch Zentrifugation verdichtet,

   und jeweils von der Mutterlösung abgetrennt werden.

6. Verfahren nach einem der Ansprüche 2, 3 oder 5, **dadurch gekennzeichnet, dass** das Trägermaterial ausgewählt wird aus mineralischen Trägermaterialien, insbesondere Quarzfasern, Silica, Glas, Aluminiumoxid, Zeolith, Titandioxid, Zirkondioxid oder Kombinationen daraus.

7. Verfahren nach einem der vorangehenden Ansprüche, **dadurch gekennzeichnet, dass** die organische Amin-Verbindung der Waschlösung im Schritt c) ausgewählt wird aus mindestens einer Verbindung der allgemeinen Formel (I),

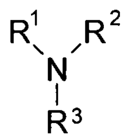

(I)

   worin $R^1$, $R^2$ und $R^3$ unabhängig voneinander stehen für ein Wasserstoffatom, eine ($C_1$ bis $C_6$)-Alkylgruppe, eine ($C_1$ bis $C_6$)-Monohydroxyalkylgruppe, eine ($C_2$ bis $C_6$)-Dihydroxyalkylgruppe, eine ($C_3$ bis $C_6$)-Trihydroxyalkylgruppe,
   mit der Maßgabe, dass das organische Amin der Formel (I) eine Molmasse von $\leq$ 500 g/mol aufweist, und mindestens einer der Reste $R^1$, $R^2$ und $R^3$ von einem Wasserstoffatom verschieden ist.

8. Verfahren nach einem der vorangehenden Ansprüche, **dadurch gekennzeichnet, dass** die organische Amin-Verbindung der Waschlösung ausgewählt wird, aus Triethylamin, Triethanolamin, 2-Amino-2-(hydroxymethyl)-propan-1,3-diol (TRIS), 2,2-Bis(hydroxymethyl)-2,2',2"-nitrilotriethanol (BIS-TRIS), 1,3-Bis[tris(hydroxymethyl)methylamino]propan (BIS-TRIS propan), oder Kombinationen davon.

9. Verfahren nach einem der vorangehenden Ansprüche, **dadurch gekennzeichnet, dass** die Waschlösung gemäß Schritt c) die besagte organische Amin-Verbindung in einer Konzentration von 200 mmol/L bis 800 mmol/L enthält.

10. Verfahren nach einem der vorangehenden Ansprüche, **dadurch gekennzeichnet, dass** das Stickstoffatom der Aminogruppe(n) der besagten Amin-Verbindung zu mindestens 50 %, bevorzugt zu mindestens 60 %, besonders bevorzugt zu mindestens 70 %, in protonierter Form vorliegt.

11. Verfahren nach einem der vorangehenden Ansprüche, **dadurch gekennzeichnet, dass** die Waschlösung gemäß Schritt c) als Lösemittel mindestens einen $C_1$-$C_6$-Monoalkohol, insbesondere Ethanol, Isopropanol oder Kombinationen davon, enthält.

12. Verfahren nach Anspruch 11, **dadurch gekennzeichnet, dass** die Waschlösung den $C_1$-$C_6$-Monoalkohol in einer Gesamtmenge von 20 Gew.-% bis 80 Gew.-% bezogen auf das Gesamtgewicht der Waschlösung enthält.

13. Verfahren nach einem der vorangehenden Ansprüche, **dadurch gekennzeichnet, dass** die Waschlösung gemäß Schritt c) einen pH-Wert im Bereich von pH 5,0 bis pH 7,0 aufweist.

14. Verfahren nach einem der vorangehenden Ansprüche, **dadurch gekennzeichnet, dass** im Rahmen der wenigstens teilweisen Entfernung jeweils bezogen auf die Gesamtmenge an Endotoxin in der flüssigen Probe wenigstens 60

Gew.-% Endotoxin, bevorzugt wenigstens 70 Gew.-% Endotoxin, entfernt werden.

**15.** Verwendung einer Waschlösung, die

(i) mindestens eine Amin-Verbindung mit mindestens zwei Kohlenstoffatomen und mit einer Molmasse von ≤ 500 g/mol und
(ii) mindestens ein von besagter Amin-Verbindung verschiedenes organisches Lösemittel, enthält und

einen pH-Wert im Bereich von pH 3,0 bis pH 8,5 aufweist,
zur wenigstens teilweisen Entfernung von Endotoxinen aus einer Nukleinsäure-haltigen Probe, **dadurch gekennzeichnet, dass** die Waschlösung die besagte organische Amin-Verbindung in einer Konzentration von 200 mmol/L bis 1000 mmol/L enthält, und
im Rahmen der wenigstens teilweisen Entfernung jeweils bezogen auf die Gesamtmenge an Endotoxin in der flüssigen Probe wenigstens 50 Gew.-% Endotoxin entfernt werden.

**16.** Verwendung nach Anspruch 15, **dadurch gekennzeichnet, dass** die Nukleinsäure und das Endotoxin auf einem festen Trägermaterial gebunden, insbesondere adsorbiert, vorliegen.

**17.** Kit zur Aufreinigung von Nukleinsäuren, insbesondere Plasmid DNA, umfassend eine Waschlösung, die

(i) mindestens eine Amin-Verbindung mit mindestens zwei Kohlenstoffatomen und mit einer Molmasse von ≤ 500 g/mol und
(ii) mindestens ein von besagter Amin-Verbindung verschiedenes organisches Lösemittel, enthält und

einen pH-Wert im Bereich von pH 3,0 bis pH 8,5 aufweist
sowie mindestens einen zusätzlichen Bestandteil, ausgewählt aus einer Bedienungsanleitung zur Durchführung eines Verfahrens nach einem der Ansprüche 1 bis 14, einem Bindepuffer und einem Lösungspuffer zum Inlösungbringen der wenigstens teilweise von Endotoxinen gereinigten Nukleinsäuren, **dadurch gekennzeichnet, dass** die Waschlösung die besagte organische Amin-Verbindung in einer Konzentration von 200 mmol/L bis 1000 mmol/L enthält, und
im Rahmen der wenigstens teilweisen Entfernung jeweils bezogen auf die Gesamtmenge an Endotoxin in der flüssigen Probe wenigstens 50 Gew.-% Endotoxin entfernt werden.

## Claims

**1.** Process for the purification of nucleic acids, in particular plasmid DNA, from a nucleic acid-containing biological sample, comprising the following steps:

a) providing a liquid sample containing nucleic acid and endotoxin each in dissolved form;
b) separation of at least nucleic acid and endotoxin from the liquid sample;
c) washing the components of the liquid sample separated in step b) for at least partial removal of the endotoxin with at least one washing solution containing

(i) at least one amine compound having at least two carbon atoms and having a molecular weight of ≤ 500 g/mol and;
(ii) at least one organic solvent different from said amine compound, and

has a pH-value in the range of pH 3.0 to pH 8.5, and;
d) dissolving of the remaining nucleic acid from the separated components washed according to step c) by means of a solution buffer and collection of the dissolved nucleic acids in a separate collecting vessel,

**characterized in that**
the washing solution according to step c) contains said organic amine compound in a concentration of 200 mmol/L to 1000 mmol/L, and
at least 50% by weight of endotoxin is removed in the course of the at least partial removal, based in each case on the total amount of endotoxin in the liquid sample.

**2.** Process according to claim 1, **characterized in that** in step b) at least nucleic acid and endotoxin are deposited by binding, in particular by adsorption or precipitation, to the surface of a solid carrier material.

**3.** Process according to claim 2, **characterized in that** the sample is mixed in step b) with a binding buffer for deposition on the carrier material.

**4.** Process according to claim 1, **characterized in that** in step b) at least the nucleic acid and the endotoxin are separated off as solids by precipitation.

**5.** Process according to claim 4, **characterized in that** the precipitated nucleic acid and the endotoxin are as a solid

(i) collected by filtration on the surface of a solid carrier material; or
(ii) compacted by centrifugation,

and in each case are separated from the mother solution.

**6.** Process according to any of claims 2, 3 or 5, **characterized in that** the carrier material is selected from mineral carrier materials, in particular quartz fibres, silica, glass, aluminumina, zeolite, titanium dioxide, zirconium dioxide or combinations thereof.

**7.** Process according to one of the preceding claims, **characterized in that** the organic amine compound of the washing solution in step c) is selected from at least one compound of the general formula (I),

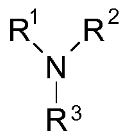

(I)

wherein $R^1$, $R^2$ and $R^3$ independently represent a hydrogen atom, a ($C_1$ to $C_6$)-alkyl group, a ($C_1$ to $C_6$)-monohydroxyalkyl group, a ($C_2$ to $C_6$)-dihydroxyalkyl group, a ($C_3$ to $C_6$)-trihydroxyalkyl group,
provided that the organic amine of formula (I) has a molecular weight of ≤ 500 g/mol, and at least one of the rests $R^1$, $R^2$ and $R^3$ is other than a hydrogen atom.

**8.** Process according to one of the preceding claims, **characterized in that** the organic amine compound of the washing solution is selected from triethylamine, triethanolamine, 2-amino-2-(hydroxymethyl)-propane-1,3-diol (TRIS), 2,2-bis(hydroxy-methyl)-2,2',2"-nitrilotriethanol (BIS-TRIS), 1,3-bis[tris(hydroxymethyl)methylamino]propane (BIS-TRIS propane), or combinations thereof.

**9.** Process according to one of the preceding claims, **characterized in that** the washing solution according to step c) contains said organic amine compound in a concentration of 200 mmol/L to 800 mmol/L.

**10.** Process according to one of the preceding claims, **characterized in that** at least 50%, preferably at least 60%, particularly preferably at least 70%, of the nitrogen atom of the amino group(s) of the said amine compound is present in protonated form.

**11.** Process according to one of the preceding claims, **characterized in that** the washing solution according to step c) contains as solvent at least one $C_1$-$C_6$ monoalcohol, in particular ethanol, isopropanol or combinations thereof.

**12.** Process according to claim 11, **characterized in that** the washing solution contains the $C_1$-$C_6$ monoalcohol in a total amount of 20 % to 80 % by weight based on the total weight of the washing solution.

**13.** Process according to one of the preceding claims, **characterized in that** the washing solution according to step c) has a pH-value in the range of pH 5.0 to pH 7.0.

**14.** Process according to one of the preceding claims, **characterized in that** at least 60% by weight of endotoxin, preferably at least 70% by weight of endotoxin, is removed in the course of the at least partial removal, based in each case on the total amount of endotoxin in the liquid sample.

**15.** Use of a washing solution containing

(i) at least one amine compound having at least two carbon atoms and having a molecular weight of ≤ 500 g/mol and
(ii) at least one organic solvent other than said amine compound, and

having a pH value in the range from pH 3.0 to pH 8.5 for at least partial removal of endotoxins from a nucleic acid-containing sample, **characterized in that** the washing solution contains the said organic amine compound in a concentration of 200 mmol/L to 1000 mmol/L, and at least 50% by weight of endotoxin is removed in the course of the at least partial removal, based in each case on the total amount of endotoxin in the liquid sample.

**16.** Use according to claim 15, **characterized in that** the nucleic acid and the endotoxin are present bound, in particular adsorbed, on a solid carrier material.

**17.** Kit for the purification of nucleic acids, in particular plasmid DNA, comprising a washing solution containing

(i) at least one amine compound having at least two carbon atoms and having a molecular weight of ≤ 500 g/mol and
(ii) at least one organic solvent other than said amine compound, and having a pH value in the range from pH 3.0 to pH 8.5

and at least one additional component selected from an operating manual for carrying out a process according to one of claims 1 to 14, a binding buffer and a solution buffer for bringing the nucleic acids at least partially purified of endotoxins into solution, **characterized in that** the washing solution contains the said organic amine compound in a concentration of 200 mmol/L to 1000 mmol/L, and
at least 50% by weight of endotoxin is removed in the course of the at least partial removal, based in each case on the total amount of endotoxin in the liquid sample.

## Revendications

**1.** Procédé de purification d'acides nucléiques, en particulier d'ADN plasmidique, à partir d'un échantillon biologique contenant un acide nucléique, comprenant les étapes suivantes:

a) fournir un échantillon liquide contenant d'acide nucléique et d'endotoxine sous forme dissoute;
b) séparation au moins d'acide nucléique et d'endotoxine de l'échantillon liquide;
c) lavage des composants de l'échantillon liquide séparés à l'étape b) pour éliminer au moins partiellement l'endotoxine avec au moins une solution de lavage contenant

(i) au moins un composé amine ayant au moins deux atomes de carbone et ayant un poids moléculaire de ≤ 500 g/mol et;
(ii) au moins un solvant organique différent dudit composé amine, et

ayant une valeur pH comprise entre 3,0 et 8,5, et;
d) mise en solution de l'acide nucléique restant des composants séparés lavés selon l'étape c) au moyen d'un tampon de solution et collecte des acides nucléiques dissous dans un récipient collecteur séparé,

**caractérisé en ce que**
la solution de lavage selon l'étape c) contient ledit composé amine organique à une concentration de 200 mmol/L à 1000 mmol/L, et
au moins 50 % en poids d'endotoxines sont éliminés au cours de l'élimination au moins partielle, sur la base de la quantité totale d'endotoxines dans l'échantillon liquide.

**2.** Procédé selon la revendication 1, **caractérisé en ce que** dans l'étape b), au moins d'acide nucléique et d'endotoxine sont déposés par liaison, en particulier par adsorption ou précipitation, sur la surface d'un matériau support solide.

**3.** Procédé selon la revendication 2, **caractérisé en ce que** l'échantillon de l'étape b) est mélangé avec un tampon de liaison pour dépôt sur le matériau support.

**4.** Procédé selon la revendication 1, **caractérisé en ce que** dans l'étape b), au moins l'acide nucléique et l'endotoxine sont séparés sous forme solide par précipitation.

**5.** Procédé selon la revendication 4, **caractérisé en ce que** l'acide nucléique précipité et l'endotoxine sont - sous forme solide -

i) recueillies par filtration à la surface d'un matériau support solide; ou
ii) compacté par centrifugation,

et á chaque fois séparé de la solution mère.

**6.** Procédé selon l'une quelconque des revendications 2, 3 ou 5, **caractérisé en ce que** le matériau de support est choisi parmi les matériaux de support minéraux, en particulier les fibres de quartz, la silice, le verre, l'alumine, la zéolite, le dioxyde de titane, le dioxyde de zirconium ou leurs combinaisons.

**7.** Procédé selon l'une des revendications précédentes, **caractérisé en ce que** le composé amine organique de la solution de lavage de l'étape c) est choisi parmi au moins un composé de formule générale (I),

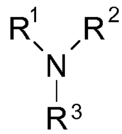

(I)

dans laquelle $R^1$, $R^2$ et $R^3$ représentent indépendamment un atome d'hydrogène, un groupe alkyle en $C_1$ à $C_6$, un groupe monohydroxyalkyle en $C_1$ à $C_6$, un groupe dihydroxyalkyle en $C_2$ à $C_6$, un groupe trihydroxyalkyle en $C_3$ à $C_6$,
à condition que l'amine organique de formule (I) ait un poids moléculaire de ≤ 500 g/mol, et qu'au moins l'un de $R^1$, $R^2$ et $R^3$ soit autre qu'un atome d'hydrogène.

**8.** Procédé selon l'une des revendications précédentes, **caractérisé en ce que** le composé amine organique de la solution de lavage est choisi parmi la triéthylamine, la triéthanolamine, 2-Amino-2-(hydroxyméthyl)propane-1,3-diol (TRIS), 2,2-bis(hydroxyméthyl)-2,2',2',2"-nitrilotriéthanol (BIS-TRIS), 1,3-bis[tris(hydroxyméthyl)méthylamino] propane (BIS-TRIS propane) ou leurs combinaisons.

**9.** Procédé selon l'une des revendications précédentes, **caractérisé en ce que** la solution de lavage selon l'étape c) contient ledit composé amine organique en une concentration de 200 mmol/L à 800 mmol/L.

**10.** Procédé selon l'une des revendications précédentes, **caractérisé en ce qu'**au moins 50%, de préférence au moins 60%, de préférence au moins 60%, de préférence au moins 70%, de l'atome d'azote du ou des groupes amino dudit composé amine est présent sous forme protonée.

**11.** Procédé selon l'une des revendications précédentes, **caractérisé en ce que** la solution de lavage selon l'étape c) contient comme solvant au moins un monoalcool en $C_1$-$C_6$, en particulier de l'éthanol, de l'isopropanol ou leurs combinaisons.

**12.** Procédé selon la revendication 11, **caractérisé en ce que** la solution de lavage contient le monoalcool en $C_1$-$C_6$ en une quantité totale de 20 à 80 % en poids par rapport au poids total de la solution de lavage.

**13.** Procédé selon l'une des revendications précédentes, **caractérisé en ce que** la solution de lavage selon l'étape c) a une valeur pH comprise entre pH 5,0 et pH 7,0.

**14.** Procédé selon l'une des revendications précédentes, **caractérisé en ce qu'**au moins 60% en poids d'endotoxine, de préférence au moins 70% en poids d'endotoxine, par rapport à la quantité totale d'endotoxine dans l'échantillon liquide, sont éliminés dans le cadre de l'élimination au moins partielle dans chaque cas.

**15.** Utilisation d'une solution de lavage contenant

(i) au moins un composé amine ayant au moins deux atomes de carbone et ayant un poids moléculaire de ≤ 500 g/mol et
(ii) au moins un solvant organique autre que ledit composé amine, et
ayant une valeur de pH comprise entre pH 3,0 et pH 8,5,

pour l'élimination au moins partielle des endotoxines d'un échantillon contenant un acide nucléique, **caractérisé en ce que** la solution de lavage contient ledit composé amine organique à une concentration de 200 mmol/L à 1000 mmol/L, et
au moins 50 % en poids d'endotoxines sont éliminés au cours de l'élimination au moins partielle, sur la base de la quantité totale d'endotoxines dans l'échantillon liquide.

**16.** Utilisation selon la revendication 15, **caractérisée en ce que** l'acide nucléique et l'endotoxine sont présents liés, en particulier adsorbés, sur un matériau support solide.

**17.** Kit pour la purification d'acides nucléiques, en particulier d'ADN plasmidique, comprenant une solution de lavage contenant

(i) au moins un composé amine ayant au moins deux atomes de carbone et ayant un poids moléculaire de ≤ 500 g/mol et
(ii) au moins un solvant organique autre que ledit composé amine, et
ayant une valeur de pH comprise entre pH 3,0 et pH 8,5, et

au moins un composant supplémentaire choisi parmi un mode d'emploi pour la mise en oeuvre d'un procédé selon l'une des revendications 1 à 14, un tampon de liaison et un tampon de solution pour mettre en solution les acides nucléiques au moins partiellement purifiés des endotoxines, **caractérisé en ce que** la solution de lavage contient ledit composé amine organique en une concentration de 200 mmol/L à 1000 mmol/L, et
au moins 50 % en poids d'endotoxines sont éliminés au cours de l'élimination au moins partielle, sur la base de la quantité totale d'endotoxines dans l'échantillon liquide.

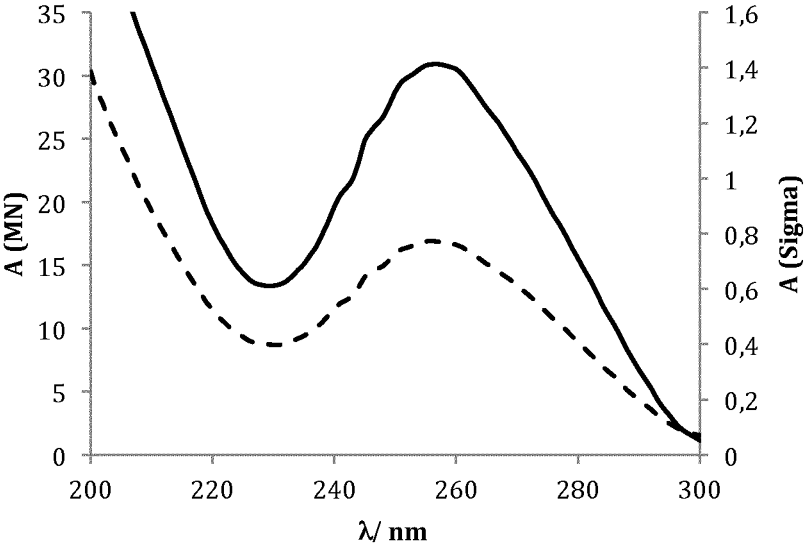
35
30
25
20
15
10
5
0
A (MN)
1,6
1,4
1,2
1
0,8
0,6
0,4
0,2
0
A (Sigma)
200
220
240
260
280
300
λ/ nm

Fig. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2007115046 A1
- WO 9521178 A1
- WO 99063076 A1
- EP 0407037 B1
- WO 2005003152 A1
- US 6699386 B2
- US 7714111 B2
- CN 103173438 B
- WO 2005111059 A2